# EUROPÄISCHE PATENTANMELDUNG

(11) **EP 3 536 351 A1**
(43) Veröffentlichungstag der Anmeldung: **11.09.2019**
(21) Anmeldenummer: 19165150.4
(22) Anmeldetag: 19.06.2013
(51) Int. Cl.: A61L 26/00

(54) **ZUSAMMENSETZUNG ZUR FÖRDERUNG DER WIEDERHERSTELLUNG VON VERLETZTEM KÖRPERGEWEBE**

(30) Priorität: 09.07.2012 DE 102012013482
(62) Teilanmeldung aus: 13737554.9
(71) Anmelder: Bitop AG, Witten 58453 (DE)
(72) Erfinder: Bilstein, Andreas, 50129 Bergheim (DE); Scherner, Olaf, 45134 Essen (DE); Lentzen, Georg, 46483 Wesel (DE)
(74) Vertreter: Schneiders & Behrendt PartmbB Rechtsanwälte - Patentanwälte

(57) **Zusammenfassung**

Die Erfindung betrifft eine Zusammensetzung, enthaltend als Wirkstoff Ectoin, Hydroxyectoin und/oder Salze, Ester oder Amide dieser Verbindungen zur Förderung der Wiederherstellung von verletztem Körpergewebe, wobei die Verletzung des Körpergewebes auf einem Ulcus, dem diabetischen Fußsyndrom, einer chronischen Wunde oder einem Dekubitus beruht.

## Beschreibung

Die Erfindung betrifft eine Zusammensetzung, mit der die Wiederherstellung von verletztem Körpergewebe gefördert werden kann.

Verletzungen von Körpergewebe können auf unterschiedliche Art und Weise zustande kommen. Insbesondere können Gewebe durch äußere Einflüsse, d. h. auf traumatische Weise entstehen. Allgemein werden derartige Gewebeverletzungen, insbesondere im Bereich der Haut oder Schleimhaut, auch als Wunde bezeichnet. Neben Gewebeverletzungen, die auf äußere Einflüsse zurückzuführen sind, sind auch nicht traumatische Verletzungen als Ulcus (Geschwür) bekannt.

Für gewöhnlich beginnt die Wiederherstellung von geschädigtem Körpergewebe auf natürliche Weise bereits kurze Zeit nach der Verletzung. Im Fall einer Wunde setzt sehr bald die Blutgerinnung ein, so dass das zerstörte Blutgefäß durch einen Blutpfropfen (Gerinnsel) verschlossen wird. In der sich anschließenden Exsudationsphase tritt Wundsekret aus, wodurch Fremdkörper und Keime aus der Wunde herausbefördert werden. Das Immunsystem sorgt für die Abtötung von Bakterien.

In einer sich anschließenden Proliferationsphase entsteht neues Bindegewebe, sodass der durch die Wunde herbeigeführte Defekt ausgefüllt wird. Schließlich wird in einer Regenerationsphase die Wunde durch Überwachsen mit Epithelzellen verschlossen, die von intaktem Epithelgewebe im Bereich der Wundränder ausgehen.

Während die natürliche Wundheilung im Regelfall und bei nicht zu großen Wunden relativ problemlos verläuft, kann es bei großflächigen Wunden unter anderem aufgrund der starken Exsudatbildung zu Komplikationen kommen. Gleiches gilt für verschiedene Ulcera. Besonders problematisch sind darüber hinaus chronische Wunden, die auf eine dauerhafte Druckbelastung zurückzuführen sind (Dekubitus) oder auch eine verzögerte Wundheilung infolge von Diabetes mellitus. Letzterer führt bei manchen Patienten zum sog. diabetischen Fußsyndrom, das für 2/3 aller Amputationen in Deutschland verantwortlich ist.

Es stellt sich somit die Aufgabe, ein Mittel zur Verfügung zu stellen, mit dem die Wiederherstellung von verletztem Körpergewebe unterstützt und gefördert werden kann.

Diese Aufgabe wird erfindungsgemäß gelöst durch eine Zusammensetzung, enthaltend als Wirkstoff Ectoin, Hydroxyectoin, Glucosylglycerol und/oder Salze, Ester oder Amide dieser Verbindungen zur Förderung der Wiederherstellung von verletztem Körpergewebe.

Bei Ectoin und Hydroxyectoin handelt es sich um Tetrahydropyrimidinderivate, die unter Stressbedingungen von extremophilen, insbesondere halophilen Mikroorganismen synthetisiert werden. Für Ectoin und Hydroxyectoin wurden bislang verschiedene Verwendungen beschrieben, beispielsweise als Moisturizer, zur Behandlung des Vascular Leak Syndroms (VLS) (DE 10 2006 056 766 A1) oder zur Behandlung von Neurodermitis (DE 103 30 243 A1). Aus der DE 100 06 578 A1 ist die Verwendung von Ectoin und seinen Derivaten zum Schutz von Biopolymeren vor dem Abbau durch degradierende Enzyme wie Proteasen, Nukleasen oder Lipasen bekannt.

Die systematische Bezeichnung für Ectoin lautet 2-Methyl-1,4,5,6-tetrahydropyrimidin-4-carbonsäure, für Hydroxyectoin 5-Hydroxy-2-methyl-1,4,5,6-tetrahydropyrimidin-4-carbonsäure.

Die Struktur des natürlichen L-Ectoins ((S)-2-Methyl-1,4,5,6-tetrahydropyrimidin-4-carbonsäure) ist im Folgenden dargestellt:

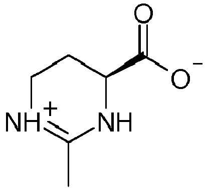

Die Struktur des natürlichen Hydroxyectoins ((4S,5S)-5-Hydroxy-2-methyl-1,4,5,6-tetrahydropyrimidin-4-carbonsäure) wird im Folgenden wiedergegeben:

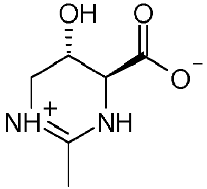

Die Verwendung der angegebenen Stereoisomere ist bevorzugt, jedoch nicht obligatorisch, d.h. auch die Verwendung anderer Stereoisomere bzw. des Racemats ist möglich.

Glucosylglycerol, genauer 2-O-α-D-Glucosylglycerol, ist als Naturstoff bekannt und wird beispielsweise von Cyanobakterien synthetisiert, denen es als osmoprotektive Substanz dient. Auf diese Weise gestattet es den Cyanobakterien das Wachstum in salzhaltigen Medien mit einer Konzentration bis zu 1,5 M NaCl. Das Molekül wird in hohen Konzentrationen im Cytoplasma akkumuliert, um auf diese Weise den aufgrund der hohen Salzkonzentration in der Umgebung vorliegenden osmotischen Druck zu verringern und die Zelle vor Wasserverlust zu schützen. Ein Beispiel ist das Cyanobakterium *Synechocystis* sp. PCC 6803.

Darüber hinaus wird das Molekül auch von Pflanzen der Gattung Myrothamnus synthetisiert. Bei diesen Pflanzen handelt es sich um wechselfeuchte Pflanzen, *Myrothamnus flabellifolia* ist ein kleiner Strauch aus dem südlichen Afrika, der auf Felsplatten wächst und bis zu 60 Zentimeter hoch wird. Die hier auftretenden mehrere Monate andauernden Dürreperioden übersteht die Pflanze unbeschadet im ausgetrockneten Zustand. Sobald es jedoch regnet, wird die Pflanze innerhalb weniger Stunden wieder grün, woraus sich auch der Beiname "Auferstehungspflanze" ergibt. Im Bereich der Kosmetik und Dermatologie ist die Verwendung von Glucosylglycerol als Moisturizer bekannt (DE 195 40 749 A1). Daneben zeigt die DE 10 2008 039 231 A1 auch die Verwendung von Glucosylglycerolen zur Steigerung der Expression von Zellschutzenzymen. Die Struktur von 2-O-α-D-Glucosylglycerol ist wie folgt:

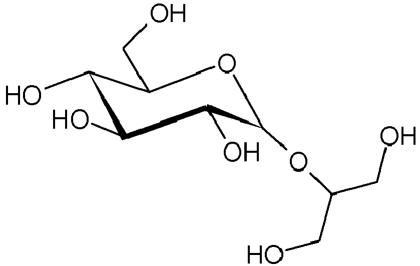

Bevorzugt handelt es sich bei dem Glucosylglycerol um das natürlich vorkommende 2-O-α-D-Glucosylglycerol, wie es beispielsweise von Cyanobakterien der Gattung *Synechocystis* akkumuliert wird. Entsprechende Wirkungen können jedoch auch bei β-glycosidischer Verknüpfung der Glucose mit dem Glycerin-Molekül oder bei Verknüpfung der Glucose mit dem Glycerin in 1-Position erwartet werden. Im Einzelnen ist somit neben der Verwendung des natürlichen Glucosylglycerols auch die Verwendung von 1-O-α-Glucosylglycerol, 1-O-β-Glucosylglycerol und 2-O-β-Glucosylglycerol in der D- und L-Konfiguration denkbar. Die einzelnen Moleküle (hier nur D-Konfiguration) sind im Folgenden dargestellt:

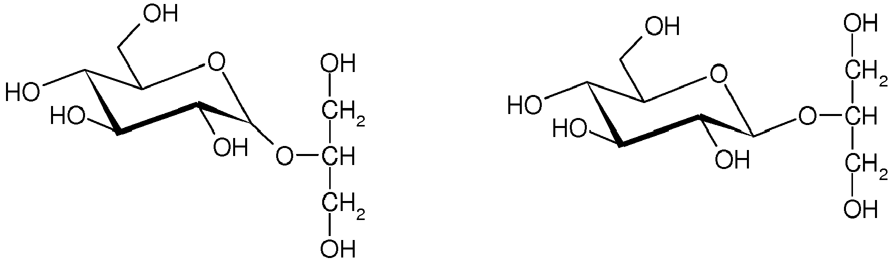

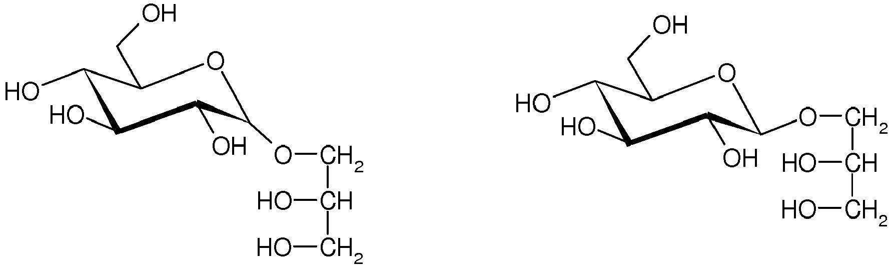

Bei dem Körpergewebe, dessen Wiederherstellung erfindungsgemäß durch die Ectoin, Hydroxyectoin oder Glucosylglycerol enthaltenden Zusammensetzungen gefördert werden kann, kann es sich insbesondere um Haut oder Schleimhaut handeln.

Im Falle von geschädigten Schleimhäuten spricht man auch von Mukositis, deren Behandlung ebenfalls unter die Förderung der Wiederherstellung von verletztem Körpergewebe gemäß Erfindung fällt. Eine Mukositis kann unterschiedliche Ursachen haben. Da Schleimhautzellen eine hohe Regenerationsrate aufweisen, tritt eine Mukositis beispielsweise häufig als Nebenwirkung im Rahmen der Krebsbehandlung durch Chemo- oder Strahlentherapie auf. Darüber hinaus sorgt ein geschwächtes Immunsystem, beispielsweise bei immunsupprimierten Patienten, für eine Zunahme von Infektionen, die ihrerseits zu Entzündungen der Schleimhaut führen können. Insbesondere können die Mundschleimhäute sowie die Schleimhäute des Verdauungssystems (Magen, Darm) betroffen sein.

Zu den Arten von Gewebeverletzungen, die mit Hilfe der erfindungsgemäßen Zusammensetzung behandelt werden können, gehören beispielsweise thermische Wunden aufgrund von Verbrennungen, Verbrühungen oder Erfrierungen, Brandwunden, Verätzungen oder Wunden, die auf ionisierende Strahlung zurückzuführen sind.

Neben Wunden lassen sich auch Prellungen mit Hilfe der erfindungsgemäßen Zusammensetzung behandeln. Bei Prellungen werden Organe oder Körperteile durch mechanische Gewalt geschädigt, ohne dass es zu einer eigentlichen Verletzung der Haut kommt. Insbesondere macht sich der Blutaustritt aus beschädigten Kapillaren in das umliegende Gewebe in Form eines Blutergusses bemerkbar.

Die erfindungsgemäße Zusammensetzung ist zur Behandlung von Geschwüren (Ulcera) geeignet. Diese können unterschiedliche Ursachen haben, beispielsweise Durchblutungsstörungen, Tumore oder Infektionen. Beispiele für mit Hilfe der erfindungsgemäßen Zusammensetzung behandelbare Geschwüre sind Ulcus cruris ("offenes Bein"), Dekubitus (Druckulcus), Malum perforans (Fußdruckgeschwür), Ulcus durum, Ulcus molle, Ulcus rodens, Ulcus corneae und andere.

Besondere Bedeutung hat die Zusammensetzung bei der Behandlung von chronischen Verletzungen, insbesondere chronischen Wunden oder chronischen Ulcera. Ein Beispiel ist das diabetische Fußsyndrom (DFS), umgangssprachlich auch als diabetischer Fuß bezeichnet. Hierbei entstehen kleinere Verletzungen, insbesondere am Fuß oder am Unterschenkel, die an sich problemlos abheilen würden, aufgrund der schlechten Wundheilung bei Diabetes-Patienten jedoch häufig von Dauer sind. Die schlechte Wundheilung ist unter anderem auf bei Diabetes-Patienten auftretende Durchblutungsstörungen zurückzuführen. Bei dem diabetischen Fußsyndrom können sich Geschwüre tief in das Körperteil hinein ausbreiten, wobei zusätzlich das Risiko besteht, dass eine Infektion mit Keimen auftritt. Die Zahl der aufgrund des diabetischen Fußsyndroms jährlich notwendigen Amputationen ist erheblich, woraus sich die Notwendigkeit der Zurverfügungstellung einer wirkungsvollen Behandlungsmöglichkeit ergibt.

Eine weitere, häufig auftretende Gewebeschädigung ist als Dekubitus (Dekubitalgeschwür, Druckgeschwür) bekannt. Dekubitus tritt insbesondere bei pflegebedürftigen Menschen auf, die ans Bett gefesselt sind und bei denen bestimmte Körperpartien einer dauernden Druckbelastung ausgesetzt sind. Überschreitet der auf die Gefäße einwirkende Druck den Kapillardruck der Gefäße, so kommt es zu einer Unterversorgung der Zellen mit Sauerstoff und Nährstoffen und damit verbunden zu einer Schädigung des Gewebes. Während Druckgeschwüre bei gesunden Menschen in der Regel nicht auftreten, da diese sich regelmäßig umlagern und gefährdete Hautstellen entlasten, sind die entsprechenden Reflexe bei pflegebedürftigen Personen teilweise nur noch eingeschränkt vorhanden. Dekubitus kann insbesondere an solchen Hautstellen auftreten, an denen Knochen der Hautoberfläche besonders nahe kommen. Schließlich besteht auch die Gefahr, dass ein offenes Dekubitalgeschwür zum Eindringen von Keimen führt. Angesichts der Zahl der pflegebedürftigen Menschen und den erheblichen Konsequenzen beim Auftreten von Dekubitus besteht gerade für diese Indikation ein besonderer Bedarf nach Behandlungsmöglichkeiten.

Weitere Körpergewebeverletzungen, bei denen die erfindungsgemäße Zusammensetzung zum Einsatz kommen kann, sind Hämorrhoidenverletzungen oder Analfissuren, die zumeist durch mechanische Belastungen hervorgerufen werden.

Die Förderung der Wiederherstellung von verletztem Körpergewebe ist auch insofern vorteilhaft, als auf diese Weise der Ausbildung von Narben vorgebeugt werden kann. Es hat sich gezeigt, dass durch die erfindungsgemäße Zusammensetzung Wunden, insbesondere der Haut, besser verheilen und aus optischen Gründen unerwünschte Narben vermieden werden.

Die erfindungsgemäße Zusammensetzung ist insbesondere für die Verabreichung auf das verletzte Körpergewebe vorgesehen, d. h. die Anwendung erfolgt insbesondere lokal bzw. topisch. Entsprechend kann die Zusammensetzung als Salbe, Creme, Lotion, Milch, Flüssigkeit, Emulsion, Mikroemulsion, Spray, Suspension, Paste, Pulver oder als sonstiger Feststoff vorliegen.

Die Zusammensetzung kann übliche Hilfsstoffe enthalten, z. B. Trägermittel, Konservierungsmittel, Bakterizide, Lösungsvermittler, Vitamine, Stabilisatoren, Substanzen zum Verhindern des Schäumens, Verdickungsmittel, Farbstoffe, oberflächenaktive Substanzen, Emulgatoren, feuchthaltende Substanzen u.ä..

Salben, Pasten, Cremes und Gele können die üblichen Trägerstoffe enthalten, z. B. tierische und pflanzliche Fette, Wachse, Paraffine, Stärke, Traganth, Zellulosederivate, Polyethylenglykole, Silikone, Bentonite, Kieselsäure, Talkum und Zinkoxid oder Gemische dieser Stoffe.

Die Zusammensetzungen können weitere Wirkstoffe enthalten, es ist jedoch insbesondere auch möglich und für die Förderung der Wiederherstellung des Körpergewebes hinreichend, Zusammensetzungen zu verwenden, die lediglich Ectoin, Hydroxyectoin und/oder Glucosylglycerol bzw. entsprechende Salze, Ester oder Amide als Wirkstoff enthalten.

Möglich ist beispielsweise die Kombination von Ectoin, Hydroxyectoin, Glucosylglycerol bzw. entsprechenden Derivaten mit einem oder mehreren Wirkstoffen ausgewählt aus: Dexpanthenol oder Derivate, Arnica montana-Extrakt (Arnica), Capsaicin, Capsicum-Extrakt, Hypericum perforatum-Extrakt (Johanniskraut), Cardiospermum halicacabum (Ballonrebe, Herzsame), Hamamelis virginiana-Extrakt (Zaubernuss), Tocopherol, Allantoin, Bisabolol, Kakao-Extrakt, Silber, Nanosilber, Mikrosilber, amorphes Silber, Silber-Salze, Zink, Zinkoxid, Calendula officinalis-Extrakt (Ringelblume), Honig und Honigextrakte, Propolis, Melilotus officinalis-Extrakt, Beinwell-Extrakt (Symphytum), Echium vulgare-Extrakt, Cumin, Angelica sinensis-Extrakt, ferulic acid (Ferularäure), hyaluronic acid (Hyaluronsäure), Aloe Vera-Extrakt, Matricaria recutita (Kamille)-Extrakt, Allium cepa-Extrakt (Zwiebel), Achillea millefolium-Extrakt (Schafgarbe), Glycyrrhiza inflata-Extrakt (Süßholz), Licochalcon A, Silikon, Urea (Harnstoff), Echinacea purpurea (Sonnenhut)-Extrakt, Chichoriensäure.

Die Konzentration an Ectoin/Hydroxyectoin, Glucosylglycerol und/oder entsprechenden Salzen, Estern oder Amiden kann insbesondere in einem Bereich zwischen 0,01 und 50 Gew.-%, insbesondere zwischen 0,5 und 20 Gew. % und besonders bevorzugt zwischen 1 und 10 Gew.-% liegen.

### Beispiel 1: Wirkung von Ectoin auf den Wundverschluss in vitro

HaCat-Zellen (humane Hautzellen) wurden auf 24-Well Kollagen-I beschichteten Platten ausplattiert. Die Zellen wuchsen bei Vollmedium (10 Gew.-% FCS = fetales Kälberserum) innerhalb von 24 h als Monolayer zusammen.

Nachdem sich dieser Zelllayer geschlossen hatte, wurde in der Mitte eine kreisrunde Verletzung mit Hilfe einer sterilen Spitze mit definiertem Durchmesser eingebracht, die im Mittel einen Durchmesser von 0,68 mm (0,363 mm²) hat.

Nach dem Setzen der Verletzung wurde das Vollmedium auf Hungermedium umgestellt, welches anstelle von 10 Gew.-% FCS (fetales Kälberserum) lediglich 0,5 Gew.-% FCS enthielt. Auf die Zellen wurden die folgenden Versuchsansätze pipettiert (pro Ansatz: n=4):
0,5 % FCS + PBS (phosphatgepufferte Salzlösung), Kontrolllösung
0,5 % FCS + 1 mM Ectoin
0,5 % FCS + 10 mM Ectoin
0,5 % FCS + 100 mM Ectoin

Die Zellen wurden über einen Zeitraum von 48 h mit den Versuchsansätzen inkubiert, wobei der Wundverschluss mittels einer Digitalkamera dokumentiert wurde. Aufnahmen wurden jeweils zu Beginn und dann in Abständen von 24 h gemacht. Die jeweils noch freien Flächen wurden ausgemessen bzw. berechnet und in mm² wiedergegeben. Das Ergebnis ist den folgenden Tabellen zu entnehmen (SD: Standardabweichung, MW: Mittelwert):

| | Fläche der Verletzung in mm² | | | | | |
|---|---|---|---|---|---|---|
| | nach 0 Stunden | | nach 24 Stunden | | nach 48 Stunden | |
| | MW | SD | MW | SD | MW | SD |
| Kontrolle | 0,363 | 0,026 | 0,212 | 0,046 | 0,080 | 0,040 |
| 1 mM Ectoin | 0,354 | 0,012 | 0,157 | 0,028 | 0,042 | 0,037 |
| 10 mM Ectoin | 0,351 | 0,033 | 0,182 | 0,034 | 0,023 | 0,029 |
| 100 mM Ectoin | 0,325 | 0,008 | 0,244 | 0,011 | 0,171 | 0,009 |

Die Ansätze, die mit 1 mM oder 10 mM Ectoin inkubiert wurden, zeigen einen eindeutigen Trend zum schnelleren Verschluss der in den Zelllayer eingebrachten kreisförmigen Verletzung im Vergleich zur Verwendung der Kontrolllösung. Lediglich bei Verwendung einer 100 mM Ectoinlösung war die Heilung der Gewebeverletzung verzögert.

### Beispiel 2: Re-Epithelialisierung bei Inkubation mit Ectoin/Hydroxyectoin

In einem konfluenten Zellrasen von ARPE-19 Zellen (adhärente retinale Pigmentepithelzellen) wurden nach 24 Stunden Inkubation mit 0, 0,1, 0,5, 1, 2,5 und 5 mM Ectoin/Hydroxyectoin in serumfreiem und serumhaltigem Medium definierte Bereiche an Zellen entfernt ("Scratchassay"). An markierten Stellen wurde die Breite des beigebrachten Spalts im Mikroskop bestimmt. Jeweils 24 und 48 Stunden danach erfolgte eine Fotodokumentation und die Spaltbreite wurde an den entsprechenden Stellen erneut bestimmt. Für jede Konzentration wurden 6 Tests durchgeführt.

Die größten Zuwachsraten der Re-Epithelialisierung waren in den ersten beiden Tagen beobachtbar. Dabei nahm die Spaltbreite in mit Ectoin/Hydroxyectoin behandelten Zellkulturen rascher ab. Hydroxyectoin war für die Re-Epithelialisierung etwas effektiver als Ectoin. Serum förderte den Spaltschluss ganz allgemein, die positiven Ergebnisse eines Zusatzes von Ectoin/Hydroxyectoin waren besonders in den serumfreien Kulturen messbar.

Der Mittelwert der Spaltbreiten nach 24 h in der nicht mit Ectoin behandelten Probe wurde auf 100 % gesetzt. Es konnten die folgenden prozentualen Spaltbreiten beobachtet werden:

| **c(Ectoin)/mM** | **Spaltbreite in %** |
|---|---|
| 0 | 100 |
| 0,1 | 100,687 |
| 0,5 | 96,434 |
| 1 | 91,632 |
| 2,5 | 93,012 |
| 5 | 88,418 |

### Beispiel 3: Wundheilungsförderung durch Hydroxyectoin und Glucosylglycerol anhand von porcinen ex-vivo Hautstanzen

Es wurde ein porcines ex-vivo Wundheilungsmodell verwendet, wie es im Patent DE 103 17 400 B4 beschrieben wird.

**Proben:**

| | Konzentration (Gew.-%) | Osmolarität |
|---|---|---|
| Hydroxyectoin I | 3,84 | 302 |
| Hydroxyectoin hyperton | 6 | 454 |
| Glucosylglycerol I | 4,76 | 305 |
| Glucosylglycerol hyperton | 7,55 | 476 |
| PBS hyperton | | 463 |
| PBS isoton | | |

| | | |
|---|---|---|
| PBS: phosphatgepufferte Salzlösung | | |

Aus den Plicae von gewaschenen und sterilisierten Schweineohren wurden Stanzen mit einem Durchmesser von 6 mm entnommen. Aus der Mitte der Stanzen wurden in einem Bereich von 3 mm die Epidermis und die obere Dermis entfernt. Sofort nach Generierung der Modelle wurden in die Wunden 5 µl der Testsubstanzen gegeben, in die Kontrollmodelle wurden 5 µl PBS appliziert. Nach 48 h (t₄₈ₕ) wurden die Modelle schockgefroren und bei -80°C aufbewahrt.

Es wurden jeweils 14 Versuchsreihen durchgeführt, so dass für jede Probe 8 bis 11 auswertbare Modelle vorhanden waren.

Anfertigung von Schnitten aus den Modellen:
Die Modelle wurden komplett in Tissue Freezing Medium (Fa. Leica, Nussloch) eingebettet und mit dem Kryostaten 6 µm dünne Schnitte angefertigt. Dabei wurde darauf geachtet, dass man sich in der Mitte des jeweiligen Modells befand. Die Schnitte wurden auf SuperFrost Objektträger gegeben, luftgetrocknet, 10 min in -20°C kaltem Aceton fixiert und bei -80°C gelagert.

Sämtliche Modelle wurden mit Hämatoxylin/Eosin gefärbt (je 2 Schnitte). Der Ablauf der Färbung war wie folgt:

| | |
|---|---|
| 1) 6 min | Inkubation mit Hämatoxylin |
| 2) kurz | spülen in Leitungswasser |
| 3) kurz | spülen in HCl-Alkohol |
| 4) 15 min | spülen in Leitungswasser fließend |
| 5) kurz | spülen in Aqua dest |
| 6) 1 min | Inkubation in Eosin (0,2%) |
| 7) kurz | Spülen in Leitungswasser |
| 8) 20 sec | Inkubation in Aqua dest |
| 9) je 20 sec 100%) | Inkubation in aufsteigender Alkoholreihe (50%, 50%, 96%, 2 x |
| 10) 20 sec | Inkubation in Xylol |
| 11) anschließend: | Schnitte trocknen und mit Eukitt eindeckeln |

Die Färbungen wurden an einem Leica Lichtmikroskop DM LS und einer Olympus Camedia Digitalkamera ausgewertet.

Statistische Auswertungen (Wundheilungsscore):
0: kein Wundheilungsfortschritt
1: kleine Wundzunge
2: große Wundzunge
3: geschlossenes Sheet
4: mehrlagig geschlossenes Sheet

Es wurde, wenn beide Wundränder auswertbar waren, das Mittel zwischen linkem und rechtem Wundrand gebildet. Die statistischen Auswertungen wurden mit Hilfe des gepaarten Student-T-Tests durchgeführt.

Auswertung:
1) Wundheilungsfortschritt absolut (alle auswertbaren aus 14 Modellen, d.h. ohne Modelle mit 2 Haarfollikeln in der Wunde oder großen Kratern), HE: Hydroxyectoin; GG: Glucosylglycerol; MW: Mittelwert; σ: Standardabweichung:

| | **MW** | **σ** | **SEM** |
|---|---|---|---|
| **HE I** | 1,92 | 0,96 | 0,30 |
| **HE hyperton** | 2,08 | 0,72 | 0,23 |
| **GG I** | 2,12 | 0,81 | 0,31 |
| **GG hyperton** | 1,66 | 0,93 | 0,30 |
| **PBS hyperton** | 1,60 | 0,84 | 0,28 |
| **PBS isoton** | 1,84 | 0,81 | 0,26 |

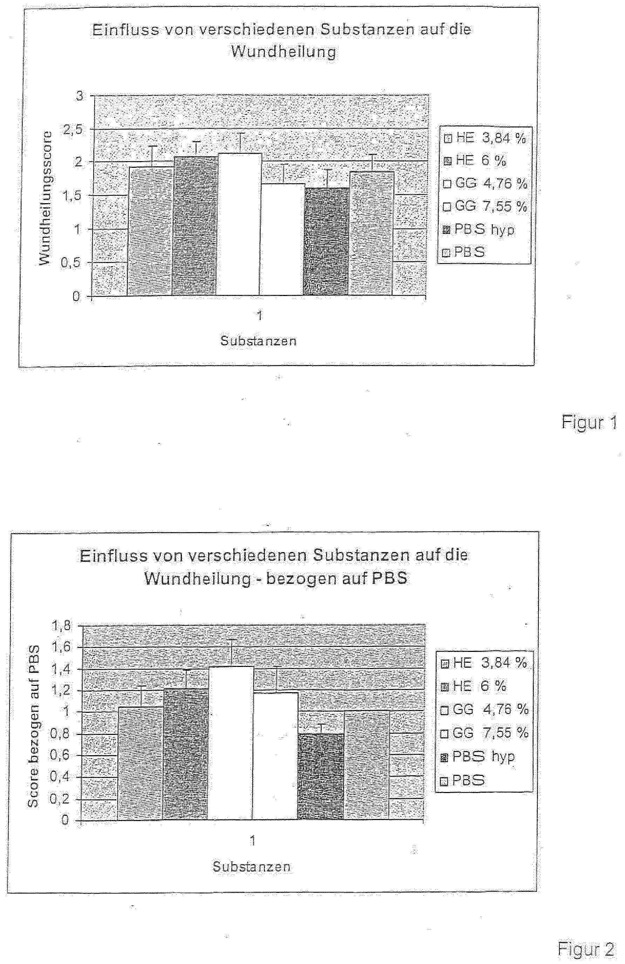

Figur 1 zeigt das Ergebnis in Form von Balkendiagrammen. Die Standardabweichungen werden als Striche über den Balken gezeigt (PBS = PBS isoton).
2) Wundheilungsfortschritt bezogen auf die PBS-Kontrolle; diese Auswertung erfolgt, um das Wundheilungspotential des einzelnen Schweins, das durch die mit PBS erzielten Werte ermittelt wird, zu berücksichtigen:

| | **MW** | **σ** | **SEM** |
|---|---|---|---|
| **HE I** | 1,05 | 0,62 | 0,20 |
| **HE hyperton** | 1,21 | 0,53 | 0,17 |
| **GG I** | 1,42 | 0,67 | 0,25 |
| **GG hyperton** | 1,17 | 0,78 | 0,25 |
| **PBS hyperton** | 0,78 | 0,30 | 0,10 |
| **PBS isoton** | 1,00 | 0 | 0 |

Figur 2 zeigt das Ergebnis in Form von Balkendiagrammen. Die Standardabweichungen werden als Striche über den Balken gezeigt (PBS = PBS isoton).

Sowohl Hydroxyectoin als auch Glucosylglycerol zeigen eine verbesserte Wundheilung.

### Gemäß vorteilhafter Ausführungsformen betrifft die Erfindung eine Zusammensetzung, wie in den nachfolgenden Positionen beschrieben:

1. Zusammensetzung, enthaltend als Wirkstoff Ectoin, Hydroxyectoin, Glucosylglycerol und/oder Salze, Ester oder Amide dieser Verbindungen zur Förderung der Wiederherstellung von verletztem Körpergewebe.
2. Zusammensetzung nach Position 1, dadurch gekennzeichnet, dass das Körpergewebe eine Haut oder Schleimhaut ist.
3. Zusammensetzung nach Position 1 oder 2, dadurch gekennzeichnet, dass die Verletzung des Körpergewebes traumatisch ist.
4. Zusammensetzung nach Position 1 oder 2, dadurch gekennzeichnet, dass die Verletzung des Körpergewebes ein Ulcus ist.
5. Zusammensetzung nach einer der Positionen 1 bis 4, dadurch gekennzeichnet, dass die Verletzung eine chronische Verletzung, insbesondere eine chronische Wunde oder ein chronisches Ulcus ist.
6. Zusammensetzung nach Position 5, dadurch gekennzeichnet, dass die Verletzung des Körpergewebes auf dem diabetischen Fußsyndrom beruht.
7. Zusammensetzung nach einer der Positionen 1 bis 5, dadurch gekennzeichnet, dass die Verletzung des Körpergewebes auf einem Dekubitus beruht.
8. Zusammensetzung nach einer der Positionen 1 bis 5, dadurch gekennzeichnet, dass die Verletzung des Körpergewebes eine Analfissur oder eine Hämorrhoidenverletzung ist.
9. Zusammensetzung nach einer der Positionen 1 bis 8, dadurch gekennzeichnet, dass das Glucosylglycerol 2-O-α-Glucosylglycerol oder 2-O-β-Glucosylglycerol ist.
10. Zusammensetzung nach Position 9, dadurch gekennzeichnet, dass das Glucosylglycerol 2-O-α-D-Glucosylglycerol ist.
11. Zusammensetzung nach einer der Positionen 1 bis 10, dadurch gekennzeichnet, dass die Zusammensetzung einen oder mehrere weitere Wirkstoffe enthält, ausgewählt aus: Dexpanthenol oder Derivate, Arnica montana-Extrakt (Arnica), Capsaicin, Capsicum-Extrakt, Hypericum perforatum-Extrakt (Johanniskraut), Cardiospermum halicacabum (Ballonrebe, Herzsame), Hamamelis virginiana-Extrakt (Zaubernuss), Tocopherol, Allantoin, Bisabolol, Kakao-Extrakt, Silber, Nanosilber, Mikrosilber, amorphes Silber, Silber-Salze, Zink, Zinkoxid, Calendula officinalis-Extrakt (Ringelblume), Honig und Honigextrakte, Propolis, Melilotus officinalis-Extrakt, Beinwell-Extrakt (Symphytum), Echium vulgare-Extrakt, Cumin, Angelica sinensis-Extrakt, ferulic acid (Ferularäure), hyaluronic acid (Hyaluronsäure), Aloe Vera-Extrakt, Matricaria recutita (Kamille)-Extrakt, Allium cepa-Extrakt (Zwiebel), Achillea millefolium-Extrakt (Schafgarbe), Glycyrrhiza inflata-Extrakt (Süßholz), Licochalcon A, Silikon, Urea (Harnstoff), Echinacea purpurea (Sonnenhut)-Extrakt, Chichoriensäure.

## Patentansprüche

1. Zusammensetzung, enthaltend als Wirkstoff Ectoin, Hydroxyectoin und/oder Salze, Ester oder Amide dieser Verbindungen zur Förderung der Wiederherstellung von verletztem Körpergewebe, **dadurch gekennzeichnet, dass** die Verletzung des Körpergewebes auf einem Ulcus, dem diabetischen Fußsyndrom, einer chronischen Wunde oder einem Dekubitus beruht.

2. Zusammensetzung nach Anspruch 1, **dadurch gekennzeichnet, dass** es sich beim Ulcus um Ulcus cruris, Dekubitus, Malum perforans, Ulcus durum, Ulcus molle, Ulcus rodens, oder Ulcus corneae handelt.

3. Zusammensetzung nach Anspruch 1 oder 2, **dadurch gekennzeichnet, dass** das Körpergewebe eine Haut oder Schleimhaut ist.

4. Zusammensetzung nach einem der Ansprüche 1 bis 3, **dadurch gekennzeichnet, dass** die Zusammensetzung einen oder mehrere weitere Wirkstoffe enthält, ausgewählt aus: Dexpanthenol oder Derivate, Arnica montana-Extrakt (Arnica), Capsaicin, Capsicum-Extrakt, Hypericum perforatum-Extrakt (Johanniskraut), Cardiospermum halicacabum (Ballonrebe, Herzsame), Hamamelis virginiana-Extrakt (Zaubernuss), Tocopherol, Allantoin, Bisabolol, Kakao-Extrakt, Silber, Nanosilber, Mikrosilber, amorphes Silber, Silber-Salze, Zink, Zinkoxid, Calendula officinalis-Extrakt (Ringelblume), Honig und Honigextrakte, Propolis, Melilotus officinalis-Extrakt, Beinwell-Extrakt (Symphytum), Echium vulgare-Extrakt, Cumin, Angelica sinensis-Extrakt, ferulic acid (Ferularäure), hyaluronic acid (Hyaluronsäure), Aloe Vera-Extrakt, Matricaria recutita (Kamille)-Extrakt, Allium cepa-Extrakt (Zwiebel), Achillea millefolium-Extrakt (Schafgarbe), Glycyrrhiza inflata-Extrakt (Süßholz), Licochalcon A, Silikon, Urea (Harnstoff), Echinacea purpurea (Sonnenhut)-Extrakt, Chichoriensäure.
